# EUROPEAN PATENT SPECIFICATION

(11) **EP 4 265 294 B1**
(45) Date of publication and mention of the grant of the patent: **08.07.2026**
(21) Application number: 21906481.3
(22) Date of filing: 09.12.2021
(51) Int. Cl.: A61N 2/00, A61N 2/10

(54) **TRANSCRANIAL MAGNETIC STIMULATOR DEVICE**
TRANSKRANIELLE MAGNETSTIMULATORVORRICHTUNG
DISPOSITIF STIMULATEUR MAGNÉTIQUE TRANSCRÂNIEN

(30) Priority: 16.12.2020 JP 2020208513
(43) Date of publication of application: 25.10.2023
(73) Proprietor: Teijin Pharma Limited, Tokyo 100-0013 (JP)
(72) Inventor: NAKAMURA, Hitoshi, Tokyo 100-0013 (JP); NANDOH, Kenji, Matsudo-shi, Chiba 270-0021 (JP)
(74) Representative: Hoffmann Eitle
(86) International application number: PCT/JP2021/045288
(87) International publication number: WO 2022/131118

(56) References cited:
- CN-A- 110 975 152
- JP-A- 2000 121 711
- JP-A- 2012 019 504
- JP-A- 2015 213 841
- JP-A- 2020 048 985
- US-A- 5 586 017
- US-A1- 2013 304 159
- US-A1- 2014 357 935
- US-A1- 2016 352 329
- US-A1- 2016 352 329
- US-A1- 2018 071 545
- US-A1- 2020 101 309
- US-B2- 7 367 936

## Description

### TECHNICAL FIELD

The present invention relates to a transcranial magnetic stimulator used for performing transcranial magnetic stimulation.

### BACKGROUND ART

Transcranial magnetic stimulation (TMS) is a method that causes a current inside a brain by electromagnetic induction, thereby stimulating neurons (see Patent Documents 1 to 5 below). With this method, a variable magnetic field is generated by applying an alternate current or a predetermined current waveform to a stimulation coil put on a head surface, an eddy current is induced by the variable magnetic field, and then, neurons can be stimulated by the eddy current. The transcranial magnetic stimulation is used for therapies of diseases, such as depression, Alzheimer's dementia, schizophrenia, neuropathic pain, and Parkinson's disease, and additionally, used for various clinical examinations and brain function studies. With the transcranial magnetic stimulation, a non-invasive magnetic stimulation can be provided to neurons inside a brain without performing a craniotomy.

Now, in a magnetic stimulator used for the conventional transcranial magnetic stimulation, an LC resonant circuit including a capacitor and a stimulation coil is used, and magnetic stimulation can be provided by generating a variable magnetic field with supply of an electric charge accumulated in the capacitor from a high-voltage power source to the stimulation coil at a required timing by turning on/off a switch disposed in the resonant circuit. Accordingly, a frequency of the current (pulse current) applied to the stimulation coil is a resonant frequency of the LC resonant circuit.

Here, in the conventional device, it is necessary to flow a pulse current of several kA in the stimulation coil for providing the magnetic stimulation with a required intensity, and the pulse voltage in the case becomes on the order of kV. Therefore, in the conventional device, a thyristor capable of dealing with high current and high voltage is used as a switching element (Patent Document 1 below). However, since the thyristor is expensive, the conventional device has a problem of the increase in manufacturing cost of the entire device.

Therefore, Patent Document 2 below proposes a technique in which an inductor with high inductance is used to reduce a current, and a relatively low-price Insulated Gate Bipolar Transistor (IGBT) is used instead of the thyristor. However, in this technique, since a voltage applied to a stimulation coil increases, and it is necessary to set a withstand voltage of the switching element to be high, there is a problem again that the cost of the switching element increases.

Patent Document 3 below proposes a technique in which a plurality of resonant circuits with stimulation coils are connected in parallel to a power source, and magnetic fields from a plurality of directions of the respective stimulation coils are combined at a single point in a deep region inside a brain, thereby allowing stimulation in the deep region inside the brain. However, in this technique, it is necessary to apply a high voltage and a high current to each of the resonant circuits. Additionally, in this technique, to achieve the stimulation to the deep region inside the brain, the stimulation coils corresponding to the respective resonant circuits are disposed at different positions and faced in various directions. Then, depending on the stimulation position assigned in the brain, any of the plurality of stimulation coils possibly needs to be disposed apart from the stimulation position. In this case, the magnetic field of the coil attenuates due to the distance, and a desired magnetic field fails to be irradiated on an irradiation position. A problem arises in that the further high voltage and high current are required to avoid this.

While Patent Document 4 below discloses a technique in which stimulation coils are connected in parallel to a power source and a charging capacitor, also in this technique, a high voltage and a high current are applied to a switching element. Therefore, this technique does not contribute to solving the above-described problem.

Patent Document 1: JP2016-67789A
Patent Document 2: JP2010-528784A
Patent Document 3: JP2010-536496A
Patent Document 4: U. S. Patent No. 7367936
Patent Document 5: WO2017/175685

US 2013/304159 A1 relates to a device for non-invasive treatment of medical conditions through delivery of energy to target tissue, comprising a source of electrical power, a magnetically permeable toroidal core, and a coil that is wound around the core. The coil and core are embedded in a continuous electrically conducting medium, which is adapted to have a shape that conforms to the contour of an arbitrarily oriented target body surface of a patient.

US 2014/357935 A1 relates to a multichannel Transcranial Magnetic Stimulation (mTMS) coil device with two or more overlap-ping coil windings within a casing. The mTMS coil device is capable of adjusting parameters of the stimulation of magnetic and induced electric fields through the selective control of the multiple coil windings within the mTMS coil device.

US 2018/071545 A1 relates to a coil apparatus for use in a transcranial magnetic stimulation apparatus in which the coil apparatus includes a coil arranged to oppose a surface of a head of a human to generate a current by an induced electric field in a magnetic stimulation-target region in a brain by electro-magnetic induction, and to stimulate neurons.

US 2020/101309 A1 relates to a magnetic field generating-apparatus for biostimulation including a C-shaped annular core having a gap-portion and coils wound around the core, wherein the core includes a pair of facing extended-portions which lie on the both sides of the gap-portion and which face to each other by extending toward the adjacent directions.

US 2016/352329 A1 relates to a magnetic field generator including a drive circuit including a MOSFET gate driver having an output terminal; a drive capacitor having a first and second terminal, a tank circuit including a tank circuit capacitor having a first and second terminal and a field-producing coil having a first and second terminal.

### DISCLOSURE OF THE INVENTION

### PROBLEMS TO BE SOLVED BY THE INVENTION

The present invention has been made based on the above-described circumstances. It is a main object of the present invention to provide a transcranial magnetic stimulator capable of providing a magnetic stimulation with a required intensity inside a brain even when a current value and a voltage value applied to magnetic stimulation coils in a plurality of resonant circuits are reduced. Another object of the present invention is to reduce a cost of an element, for example, a switching element, used for the resonant circuit by reducing the current value and the voltage value in the resonant circuit.

### SOLUTIONS TO THE PROBLEMS

The invention solves the above-described problems with a transcranial magnetic stimulator as defined in the appended claims. Embodiments, examples or aspects in the following disclosure which do not fall under the scope of the claims are presented for illustration purposes only and do not form part of the invention.

### EFFECTS OF THE INVENTION

According to the present invention, even when a current value and a voltage value applied to magnetic stimulation coils in a plurality of resonant circuits are reduced, by overlapping magnetic fluxes of the respective magnetic stimulation coils, a magnetic stimulation with a required intensity can be provided inside a brain. Accordingly, the current value and the voltage value of the resonant circuit can be reduced to be low, and consequently, the withstand voltage of the element, for example, a switching element used for the resonant circuit can be reduced. This also allows the reduction of the device cost.

### BRIEF DESCRIPTION OF THE DRAWINGS

Fig. 1 is a block diagram illustrating a schematic configuration of a transcranial magnetic stimulator according to a first embodiment of the present invention.
Fig. 2 is a circuit diagram for describing a circuit configuration in the transcranial magnetic stimulator of Fig. 1.
Fig. 3 is a schematic perspective view for describing an exemplary configuration of magnetic stimulation coils used for the circuit of Fig. 2.
Fig. 4 is an explanatory view for describing a stacked state of the magnetic stimulation coils disposed in an up-down direction, and a drawing corresponding to an end surface taken along the line A-A of Fig. 3.
Fig. 5(a) illustrates a time waveform of a pulse voltage applied to the magnetic stimulation coil, and Fig. 5(b) illustrates a time waveform of a pulse current flowing in the magnetic stimulation coil.
Fig. 6(a) is an explanatory view for describing an exemplary pattern of a magnetic stimulation to a living body, and Fig. 6(b) is an enlarged view of a part of Fig. 6(a).
Fig. 7 is an explanatory view for describing a configuration of magnetic stimulation coils used for a transcranial magnetic stimulator according to a second embodiment of the present invention, and an end view of a part corresponding to Fig. 4.
Fig. 8 is an explanatory view for describing a configuration of magnetic stimulation coils used for a transcranial magnetic stimulator according to a third embodiment of the present invention, and an end view of a part corresponding to Fig. 4.
Fig. 9 is an explanatory view illustrating an exemplary configuration of a multicore wire.
Fig. 10 is a circuit diagram in a transcranial magnetic stimulator according to a fourth embodiment of the present invention.
Fig. 11 is a drawing illustrating time waveforms of a voltage of a resonant capacitor and a current flowing in a magnetic stimulation coil.
Fig. 12 is a circuit diagram in a transcranial magnetic stimulator according to a fifth embodiment of the present invention.
Fig. 13 is a circuit diagram in a transcranial magnetic stimulator according to a sixth embodiment of the present invention.
Fig. 14 is a circuit diagram in a transcranial magnetic stimulator according to a seventh embodiment of the present invention.
Fig. 15 is a circuit diagram illustrating an example of a phase adjustment circuit used in the circuit of Fig. 14.
Fig. 16 is a circuit diagram illustrating another example of the phase adjustment circuit used in the circuit of Fig. 14.
Fig. 17 is a circuit diagram illustrating an example of a phase adjustment circuit used in a transcranial magnetic stimulator according to a seventh embodiment of the present invention.
Fig. 18 is a circuit diagram in a transcranial magnetic stimulator according to a ninth embodiment of the present invention.
Fig. 19 is a circuit diagram illustrating an example of a phase adjustment circuit used in the circuit of Fig. 18.
Fig. 20 is a circuit diagram illustrating an example of a phase adjustment circuit used in a transcranial magnetic stimulator according to a tenth embodiment of the present invention.
Fig. 21(a) illustrates an example of a current waveform applied to a magnetic stimulation coil in the circuit of Fig. 20, and Fig. 21(b) illustrates an exemplary waveform of an electric field generated by the current of Fig. 21(a).

### DESCRIPTION OF PREFERRED EMBODIMENTS

The following describes a transcranial magnetic stimulator (hereinafter simply referred to as a "stimulator" or a "device" in some cases) according to a first embodiment of the present invention with reference to the accompanying drawings. The device of the embodiment applies a variable magnetic field to an inside of a living body, thereby stimulating the living body, especially inside a brain.

### (Configuration of The Embodiment)

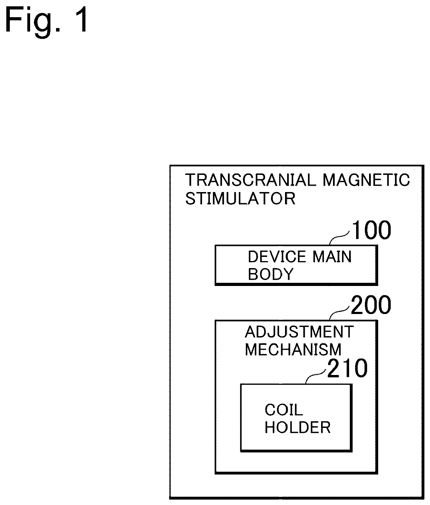

First, an example of a schematic configuration of the device according to the embodiment will be described with reference to Fig. 1. This device includes a device main body 100 and an adjustment mechanism 200. The device main body 100 supports the adjustment mechanism 200, and includes main equipment, such as a power source 3 described later. The adjustment mechanism 200 is configured to adjust a position of a coil holder 210 that holds magnetic stimulation coils 11, 12 described later, thereby providing a magnetic stimulation at a predetermined position of a head of a subject (not illustrated) seated on an appropriate chair (not illustrated). Since the device can have the overall configuration similar to a conventional one, the description in more detail will be omitted.

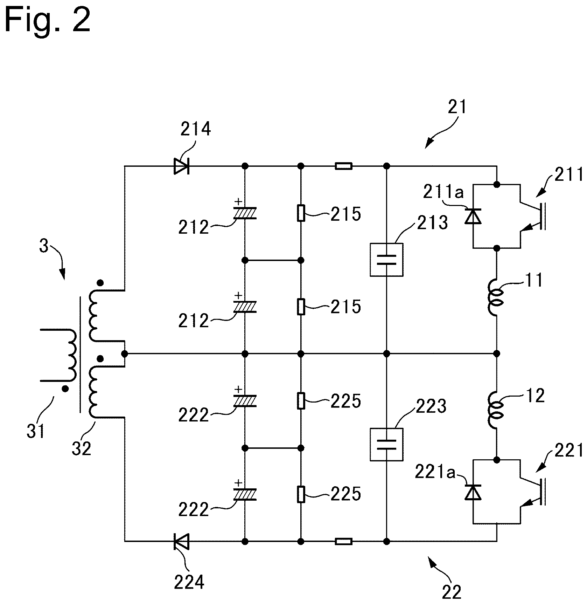

Next, with reference to Fig. 2, an exemplary configuration of a circuit for driving the magnetic stimulation coil 11, 12 in the device of this embodiment will be described. That is, the device includes a plurality of resonant circuits 21, 22 and a power source 3 as a basic configuration. The plurality of resonant circuits 21, 22 apply respective pulse currents to the plurality of magnetic stimulation coils 11, 12 for stimulating the living body by applying variable magnetic fields to an inside of a living body (specifically, inside brain) of a subject, thereby generating a variable magnetic field. The power source 3 supplies an electric power to the plurality of resonant circuits 21, 22.

### (Resonant Circuit)

The plurality of resonant circuits 21, 22 are connected in parallel to the power source 3, and accordingly, the plurality of magnetic stimulation coils 11, 12 are also connected in parallel to the power source 3.

The plurality of resonant circuits 21, 22 include a plurality of switching elements 211 and 221 that control application timings of the pulse currents to the magnetic stimulation coils 11, 12, charging capacitors 212, 222 that accumulate electric charges supplied from the power source 3, resonant capacitors 213, 223 interposed in parallel to the magnetic stimulation coils 11, 12, rectifier diodes 214, 224, and resistors 215, 225 interposed in parallel to the charging capacitors 212, 222.

In the device of the embodiment, IGBTs are used as the switching elements 211, 221, and Free Wheeling Diodes (FWD) 211a, 221a for load current commutation are connected in parallel to the IGBTs. The switching elements 211, 221 are configured to perform an on/off operation at a predetermined timing by a control device (not illustrated). The operation of the switching elements 211, 221 will be described later in detail.

The charging capacitors 212, 222 are each implemented by connecting two capacitors in series, and this is intended to improve a withstand voltage of the capacitor. The resistors 215, 225 are intended to adjust voltages applied to the capacitors connected in series.

The resonant capacitors 213, 223 constitute parallel resonant circuits resonating at a predetermined frequency together with the magnetic stimulation coils 11, 12 connected in parallel.

The resonant circuits 21, 22 of the embodiment are configured to apply required voltages to the parallel resonant circuits (that is, resonant circuits including the magnetic stimulation coils 11, 12 and the resonant capacitors 213, 223) via the switching elements 211, 221 by electric potential differences accumulated in the charging capacitors 212, 222.

### (Magnetic Stimulation Coil)

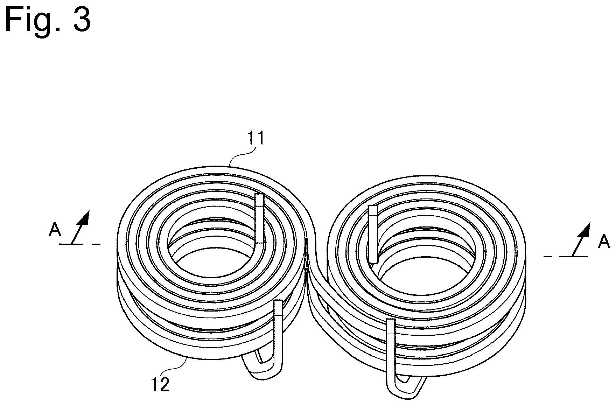

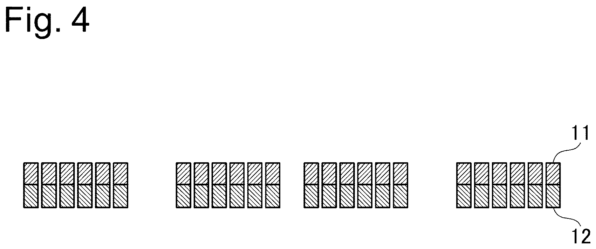

The plurality of magnetic stimulation coils 11, 12 are formed in approximately the same shape, and are adjacently disposed such that directions of magnetic fluxes generated by the applied pulse currents are matched (see Fig. 3 and Fig. 4). That is, the plurality of magnetic stimulation coils 11, 12 are disposed to be stacked such that their axial centers are approximately matched. More specifically, one of the plurality of magnetic stimulation coils 11, 12 is disposed as an upper coil 11, and the other is disposed as a lower coil 12.

The upper coil 11 and the lower coil 12 are disposed to be stacked such that a bottom surface of the upper coil 11 is overlapped with an upper surface of the lower coil 12 in a cross-sectional view of at least a part thereof (see Fig. 4). In Fig. 3, for visibility, a state where the upper coil 11 is slightly spaced from the lower coil 12 is illustrated. While both of the coils 11, 12 in the embodiment are what is called figure-eight coils, a coil having another shape may be used.

### (Power Source)

As the power source 3, in this embodiment, a step-up transformer including a primary side coil 31 and a secondary side coil 32 is used. The primary side coil 31 is connected to, for example, a commercial AC power supply, and is configured to be supplied with a required electric power. The secondary side coil 32 is what is called a center-tapped coil, and is configured to supply respective required electric powers to the resonant circuits 21, 22 in one side and the other side across the center tap.

### (Operation in The Embodiment)

Next, an operation of the device of the embodiment having the above-described configuration will be described. Here, since both of the plurality of resonant circuits operate basically similarly, one resonant circuit 21 will be basically described below as an example.

First, assume that the switching element 211 is OFF in the initial state. In this state, when a predetermined voltage is supplied from the power source 3, the voltage rectified by the rectifier diode 214 is applied to the charging capacitors 212, and the electric charge is accumulated. Then, when the switching element 211 is turned ON by an input signal from the control device (not illustrated) at a predetermined timing, the current from the charging capacitor 212 flows in the upper coil (one magnetic stimulation coil) 11 at a resonant frequency of an LC parallel resonant circuit including the magnetic stimulation coil 11 and the resonant capacitor 213. Subsequently, when the switching element 211 is turned OFF at a predetermined timing, the status returns to the initial state. In the following, the similar operation is repeated.

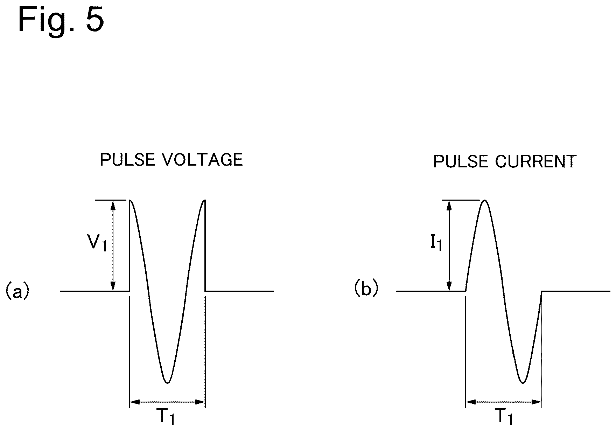

Fig. 5 illustrates an example of a voltage waveform and a current waveform applied to the coil 11. These are both sine waves, and their frequencies are determined depending on the resonant frequency of the resonant circuit. The voltage waveform and the current waveform are out of phase with each other by 90°. A time period in which the switching element 211 is ON is assumed to be T1. The time period T1 is, for example, 200 to 300 µs. However, the time period can be changed depending on the usage of the magnetic stimulation as necessary. In this example, T1 matches a cycle of the resonant frequency. While it is assumed that, in the device of this embodiment, a maximum applied voltage V₁ in the positive direction to the coil 11 is 1.8 kV, and a maximum current I₁ flowing in the positive direction is 7 kA, this is merely one example, and the maximum applied voltage V₁ can be adjusted depending on the magnitude of the required stimulation.

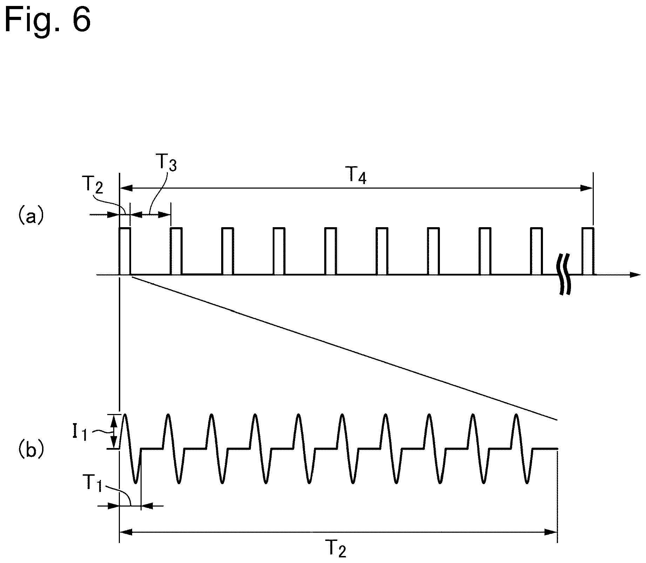

Fig. 6 illustrates an example of a treatment pattern in this embodiment. In this example, the treatment is performed during a time period (treatment time) T2 (see Fig. 6(a)), and the treatment is stopped during a next time period (downtime) T3. This operation is periodically performed. A whole treatment time T4 is, for example, from 30 minutes to 40 minutes. In one treatment time T2, by turning on/off the switching element 211, for example, a pulse current of 10 pulses per second (that is, 10 pps) is applied to the coil 11, and a variable magnetic field can be applied to the living body from the coil 11. For example, when a variable magnetic field of 3000 pulses is applied to the living body, the treatment time T4 in this example is 37.5 minutes. Obviously, this values are merely one example, and can be changed as necessary. A ratio (duty ratio) between the treatment time T2 and the downtime T3 also can be appropriately set depending on the usage. The operation of the resonant circuit 22 is similar to the above-described operation of the resonant circuit 21.

In this embodiment, since the plurality of magnetic stimulation coils 11, 12 are formed in approximately the same shape, approximately the same inductance characteristics can be obtained. Therefore, the phases of fluctuation of the magnetic fields generated from the respective coils in the resonance become approximately the same phase. Then, since the magnetic stimulation coils are adjacently disposed such that the directions of the magnetic fluxes generated by the pulse currents are matched, by mutually overlapping the generated magnetic fluxes, the magnetic flux increased to a required degree in intensity can be applied to the living body. Accordingly, there is an advantage that the current and the voltage to be applied per one of the magnetic stimulation coils 11, 12 can be reduced to be low. Then, since a low-price element, for example, an IGBT as a general-purpose product can be used as the switching elements 211, 221, an advantage of allowing the reduction of the manufacturing cost of the device to be low also can be provided.

In this embodiment, since the currents flowing in the magnetic stimulation coils 11, 12 can be reduced, the heat generation amount per one of the magnetic stimulation coils can be reduced. Accordingly, cooling measures can be facilitated, thus providing an advantage that the cooling mechanism can be simplified or eliminated.

Furthermore, since the maximum voltage and the maximum current can be reduced in each of the resonant circuits, a generated electromagnetic noise can be reduced, and as a result, measures against noise can be simplified. Insulation measures also can be simplified.

### (Second Embodiment)

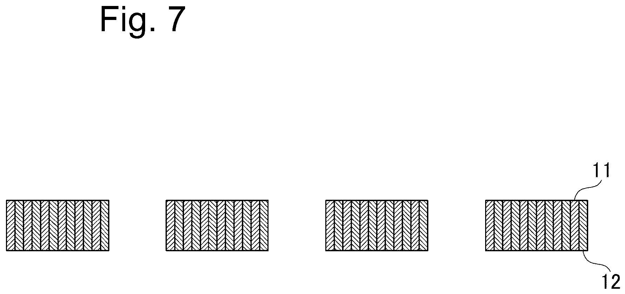

Next, a transcranial magnetic stimulator according to the second embodiment of the present invention will be described with reference to Fig. 7. In the description of the second embodiment, for the components basically in common with the device according to the first embodiment, the same reference numerals are used, thereby avoiding the overlapping description.

In the first embodiment, as the plurality of magnetic stimulation coils 11, 12, the upper coil 11 and the lower coil 12 are used. In contrast, in the second embodiment, as illustrated in Fig. 7, the plurality of magnetic stimulation coils 11, 12 are disposed such that wound wires of these coils 11, 12 are adjacent in a right-left direction (direction perpendicular to the axial center). That is, the magnetic stimulation coils 11, 12 of this embodiment are dual spiral coils concentrically stacked in a radial direction. The magnetic stimulation coils 11 and 12 are mutually insulated.

Since other configurations and the advantage of the second embodiment are similar to the first embodiment, the further detailed description will be omitted.

### (Third Embodiment)

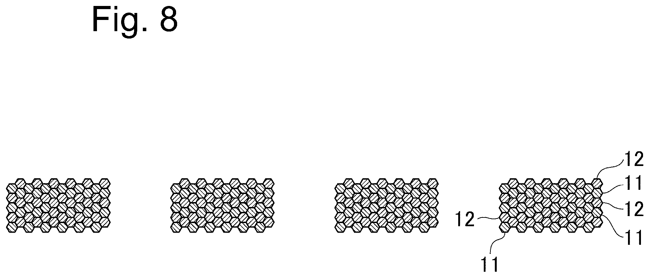

Next, a transcranial magnetic stimulator according to the third embodiment of the present invention will be described with reference to Fig. 8. In the description of the third embodiment, for the components basically in common with the device according to the first embodiment, the same reference numerals are used, thereby avoiding the overlapping description.

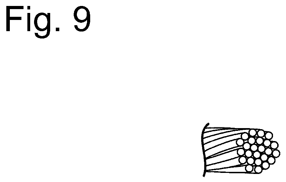

While in the first embodiment, as the plurality of magnetic stimulation coils 11, 12, the upper coil 11 and the lower coil 12 are used, in the third embodiment, as illustrated in Fig. 8, the respective wound wires of the plurality of magnetic stimulation coils 11, 12 are mutually twisted, and form a multicore wire. That is, in this embodiment, a group of core wires in the multicore wire (what is called a litz wire) constitutes one coil, and the other group of core wires constitutes the other coil. Obviously, outer peripheral surfaces of the respective core wires are insulated. In the example of Fig. 8, the core wires in the even-numbered layers from the top constitute the one magnetic stimulation coil 11, and the core wires in the odd-numbered layers constitute the other magnetic stimulation coil 12. Fig. 9 illustrates a specific example of the multicore wire. In Fig. 9, a cross-sectional shape of the whole multicore wire is a circular shape. A cross-sectional shape of the individual core wire is also a circular shape.

Since other configurations and the advantage of the third embodiment are similar to the first embodiment, the further detailed description will be omitted.

### (Fourth Embodiment)

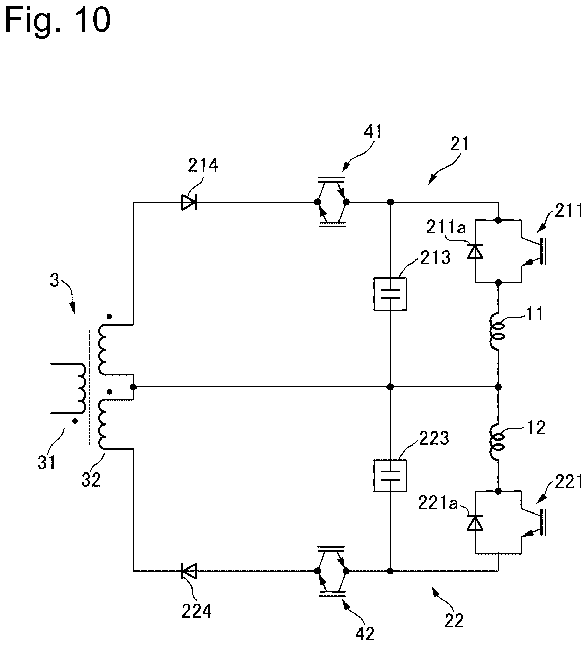

Next, a transcranial magnetic stimulator according to the fourth embodiment of the present invention will be described with reference to Fig. 10. In the description of the fourth embodiment, for the components basically in common with the device according to the first embodiment, the same reference numerals are used, thereby avoiding the overlapping description. In the fourth embodiment, the charging capacitors 212, 222 in the first embodiment are omitted, and the resonant capacitors 213, 223 double as the charging capacitors. That is, the resonant circuits 21, 22 in the fourth embodiment are LC resonant circuits including the magnetic stimulation coils 11, 12 and the resonant capacitors 213, 223.

In the fourth embodiment, between the resonant capacitors 213, 223 of the resonant circuits 21, 22 and the power source 3, second switching elements 41, 42 (see Fig. 10) that block the connection between the resonant capacitors 213, 223 and the power source 3 during the discharge of the resonant capacitors 213, 223 are disposed.

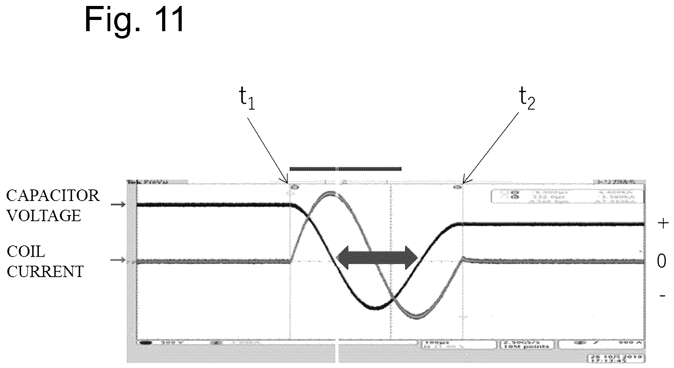

The operation of the second switching elements 41, 42 will be described further with reference to Fig. 11. This drawing illustrates an example of temporal changes of the voltages of the resonant capacitors 213, 223 and the currents flowing in the magnetic stimulation coils 11, 12 when the switching elements 211, 221 turn ON at a time t1, and turn OFF at a time t2. The voltages of the resonant capacitors 213, 223 decrease from the time t1, become negative voltages at a certain time point, and then, return to values close to the original voltage. Here, during the negative voltages (that is, during discharge of the charging voltage), parts of the currents to be flowed in the magnetic stimulation coils 11, 12 leak to the power source 3 side, and these become a loss. Then, it takes a time to recharge the resonant capacitors 213, 223, and this interferes with providing higher frequency of the magnetic stimulation pulse applied to a subject. Therefore, in this embodiment, by disposing the second switching elements 41, 42 to block the connection between the resonant capacitors 213, 223 and the power source 3 during the discharge of the resonant capacitors 213, 223, the leakage of the electric charge is reduced, thus allowing the improvement of the energy efficiency of the device. Accordingly, the leakage current in the power source 3 direction can be avoided, and consequently, the recharging time of the resonant capacitors 213, 223 is reduced, and the pulse cycle of the variable magnetic field can be reduced (that is, higher frequency can be provided). Additionally, avoiding the leakage current reduces the power consumption, thereby allowing the device to avoid the heat generation and a heat insulation structure to be simplified. In this embodiment, since a bidirectional switch is used for the second switching elements 41, 42, there is an advantage that a path for causing the charging capacitor to absorb an overvoltage generated on the wiring inductance of the device can be ensured.

Since other configurations and the advantage of the fourth embodiment are similar to the first embodiment, the further detailed description will be omitted.

### (Fifth Embodiment)

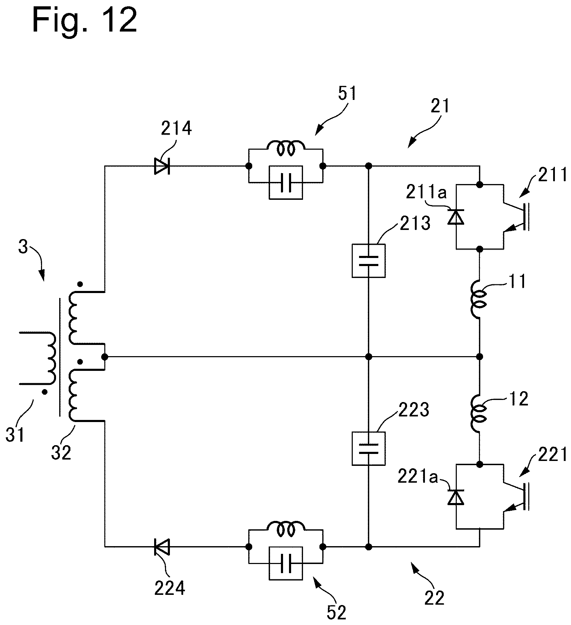

Next, a transcranial magnetic stimulator according to the fifth embodiment of the present invention will be described with reference to Fig. 12. In the description of the fifth embodiment, for the components basically in common with the device according to the fourth embodiment, the same reference numerals are used, thereby avoiding the overlapping description.

In the fifth embodiment, between the resonant capacitors 213, 223 and the power source 3, resonant impedance circuits 51, 52 including LC parallel resonant circuits are interposed. The resonant impedance circuits 51, 52 are configured to resonate at the resonant frequencies of the resonant circuits 21, 22, thereby acting as resistance components (infinite impedance in principle) higher than resistance components in non-resonance.

As described in the fourth embodiment, during the discharge of the resonant capacitors 213, 223, parts of the electric charges to be flowed in the coils 11, 12 leak to the power source 3 side. Therefore, in the fifth embodiment, the resonant impedance circuits 51, 52 suppress the current to the power source 3 side, thereby allowing the improvement of the energy efficiency of the device. Additionally, in the fifth embodiment, since the leakage current can be efficiently suppressed by only passive elements without using active elements, not only the device cost can be reduced, but also the reliability and the durability of the device can be improved. Here, while using resistive elements instead of the resonant impedance circuit can slightly reduce the leakage current, using the resonant impedance circuit provides an advantage of the high suppression effect to the leakage current.

Since other configurations and the advantage of the fifth embodiment are similar to the fourth embodiment, the further detailed description will be omitted.

### (Sixth Embodiment)

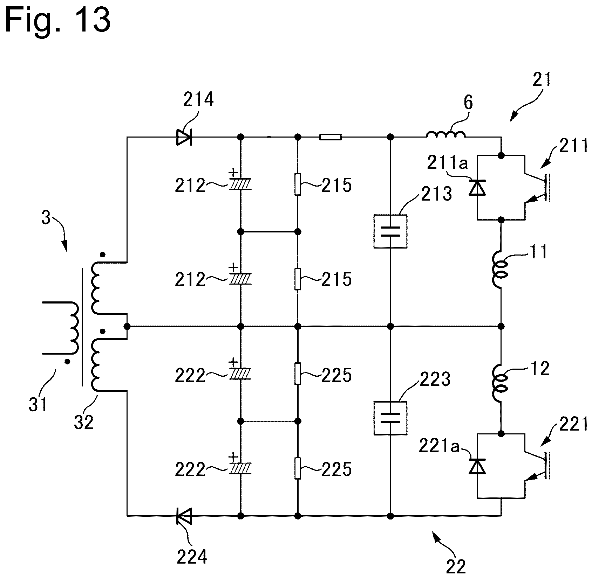

Next, a transcranial magnetic stimulator according to the sixth embodiment of the present invention will be described with reference to Fig. 13. In the description of the sixth embodiment, for the components basically in common with the device according to the first embodiment, the same reference numerals are used, thereby avoiding the overlapping description.

In the sixth embodiment, a synchronization adjustment circuit 6 for synchronizing the resonant frequencies between the respective resonant circuits is interposed in any or both of the resonant circuits. Specifically, in the example of Fig. 13, as the synchronization adjustment circuit 6, a minute inductance component interposed in the resonant circuit 21 to be in series with the magnetic stimulation coil 11 is used.

According to the sixth embodiment, by the fine adjustment of the inductance component of the resonant circuit 21, the resonant frequency of the resonant circuit 21 can be adjusted to synchronize the resonant frequencies of the respective resonant circuits. That is, with the device of this embodiment, the phases of the pulsed magnetic fluxes from the magnetic stimulation coils can be more accurately matched. Consequently, the maximum voltages and the maximum currents of the respective resonant circuits can be more suppressed.

The synchronization adjustment circuit 6 may adjust another component (for example, a capacitance component) that determines the resonant frequency. The synchronization adjustment circuit 6 may be configured to be interposed in another resonant circuit other than the resonant circuit 21, and adjust the resonant frequency of the resonant circuit.

Since other configurations and the advantage of the sixth embodiment are similar to the first embodiment, the further detailed description will be omitted.

### (Seventh Embodiment)

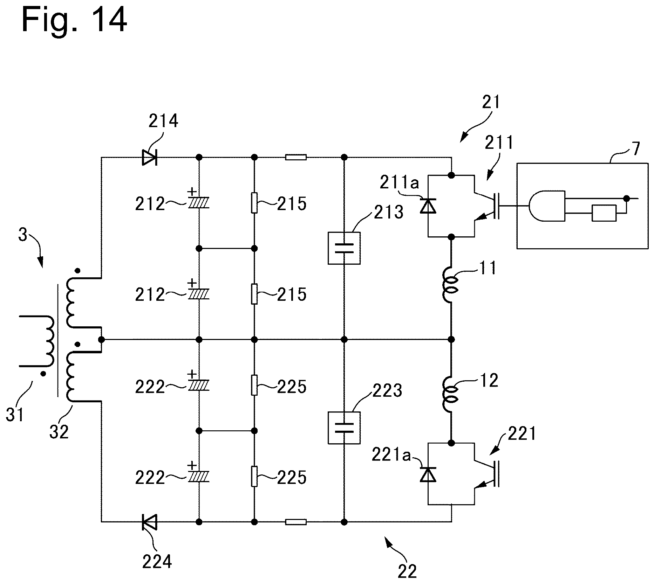

Next, a transcranial magnetic stimulator according to the seventh embodiment of the present invention will be described with reference to Fig. 14. In the description of the seventh embodiment, for the components basically in common with the device according to the first embodiment, the same reference numerals are used, thereby avoiding the overlapping description.

The device of the seventh embodiment includes a phase adjustment circuit 7 for matching phases of respective resonant currents generated in the resonant circuits 21, 22. The phase adjustment circuit 7 performs the adjustment so as to match the generation timings of the respective resonant currents generated in the resonant circuits 21, 22, thereby matching the phases of the resonant currents.

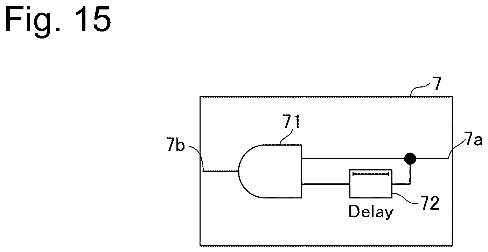

Fig. 15 illustrates an example of the phase adjustment circuit 7. The phase adjustment circuit 7 illustrated in Fig. 15 includes an AND gate 71 and a delay circuit 72. To one input terminal 7a of the AND gate 71, an input signal (ON signal) to the switching element 211 is input from the control device (not illustrated). The delay circuit 72 is configured to delay a signal to the other input terminal of the AND gate 71 corresponding to a phase shift between the resonant current of the resonant circuit 21 and the resonant current of the resonant circuit 22. An output terminal 7b of the AND gate 71 is connected to a gate of the switching element 211.

The device of this embodiment can adjust the generation timing of the resonant current by delaying the input signal to the switching element 211. That is, the adjustment can be performed in the direction decreasing the difference of the resonance start timing. Accordingly, the phases of the resonant currents generated in the resonant circuits, that is, the phases of the pulsed magnetic fluxes generated from the magnetic stimulation coils 11, 12 can be more accurately matched (that is, the difference can be decreased). Consequently, the maximum voltages and the maximum currents of the respective resonant circuits can be more suppressed.

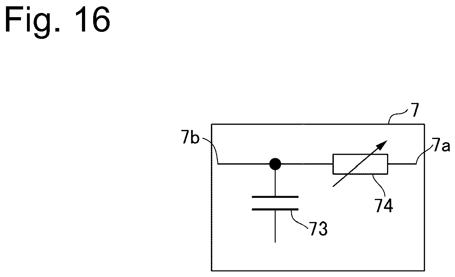

As the phase adjustment circuit 7, not limited to the example of Fig. 15, another configuration capable of adjusting the phase of the resonant current can be used. For example, as illustrated in Fig. 16, as the phase adjustment circuit 7, a capacitance element 73 and a variable resistor 74 may be used. To an input terminal 7a of this phase adjustment circuit 7, the input signal (ON signal) from the control device is input, and an output terminal 7b is connected to the gate of the switching element 211. A delay time of the circuit of Fig. 16 is determined by constants that are Cies (input capacitance) and Vth (threshold voltage) of the gate of the switching element 211, a capacitance C of the capacitance element 73 of the phase adjustment circuit 7, and a resistance value R of the variable resistor 74. Accordingly, the delay time can be controlled by adjusting the resistance value R of the variable resistor 74.

The phase adjustment circuit 7 may be connected to the resonant circuit 22 instead of the resonant circuit 21. The phase adjustment circuits 7 connected to the respective resonant circuits 21, 22 may be different each other.

Since other configurations and the advantage of the seventh embodiment are similar to the first embodiment, the further detailed description will be omitted.

### (Eighth Embodiment)

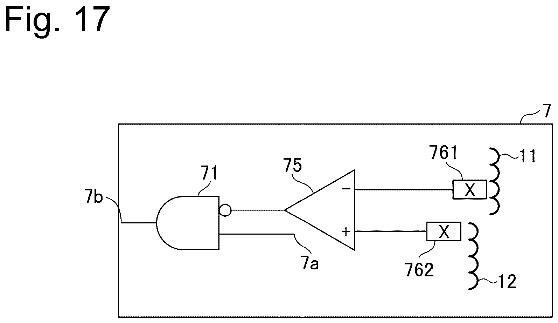

Next, a transcranial magnetic stimulator according to the eighth embodiment of the present invention will be described with reference to Fig. 17. In the description of the eighth embodiment, for the components basically in common with the device according to the seventh embodiment, the same reference numerals are used, thereby avoiding the overlapping description.

The eighth embodiment describes a further specific example of the phase adjustment circuit 7 described in the seventh embodiment. The phase adjustment circuit 7 includes a difference amplifier 75 that outputs a signal to an inverting input of an AND gate 71, and Hall elements 761, 762 that output signals to inputs of the difference amplifier 75. To another input terminal 7a of the AND gate 71, a switching signal from the control device (not illustrated) is input. The Hall element 761 is disposed in a proximity of the magnetic stimulation coil 11, and configured to detect an intensity of a magnetic field generated from the magnetic stimulation coil 11 as a voltage value. Similarly, the Hall element 762 is disposed in a proximity of the magnetic stimulation coil 12, and configured to detect an intensity of a magnetic field generated from the magnetic stimulation coil 12 as a voltage value.

In the device of the eighth embodiment, when there is no difference between the signals from the Hall elements 761, 762 (that is, when the phases of the magnetic field intensities are matched), an IGBT signal (that is, a switching signal) from the control device is directly input to the switching element 211. When there is a difference between the signals from the Hall elements 761, 762 (that is, when the phases of the magnetic field intensities are shifted), the IGBT signal (that is, a switching signal) from the control device is not input to the switching element 211, and becomes a state of hold. This allows automatically adjusting the resonance start timing in the resonant circuit 21.

Since other configurations and the advantage of the eighth embodiment are similar to the seventh embodiment, the further detailed description will be omitted.

### (Ninth Embodiment)

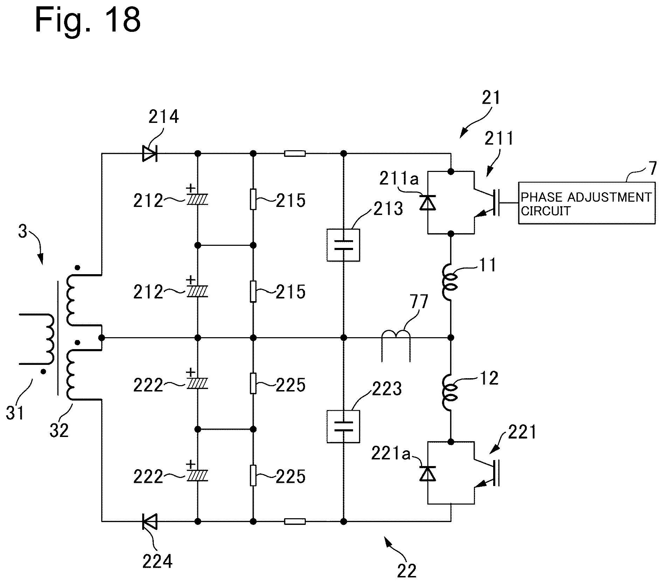

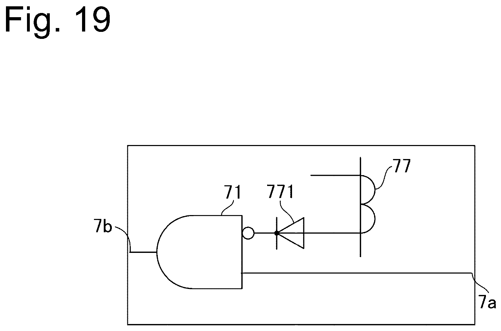

Next, a transcranial magnetic stimulator according to the ninth embodiment of the present invention will be described with reference to Fig. 18 and Fig. 19. In the description of the ninth embodiment, for the components basically in common with the device according to the seventh embodiment, the same reference numerals are used, thereby avoiding the overlapping description.

The ninth embodiment describes a further specific example of the phase adjustment circuit 7 described in the seventh embodiment. A current sensing element 77 is interposed in a common wiring part of the resonant circuits 21, 22 of the ninth embodiment (see Fig. 18). The current sensing element 77 detects a current value of the common wiring. In the phase adjustment circuit 7 of the ninth embodiment, a signal from the current sensing element 77 is input to an inverting input of an AND gate 71 via a rectifier diode 771 (see Fig. 19).

In the device of the ninth embodiment, when there is no phase difference between the respective resonant currents flowing in the resonant circuits 21, 22, the current does not flow in the common wiring part of these circuits. Accordingly, an IGBT signal (that is, a switching signal) from the control device is directly input to the switching element 211. When there is a phase difference between the respective resonant currents flowing in the resonant circuits 21, 22, the current corresponding to the phase difference flows in the common wiring part of these circuits. Then, the IGBT signal (that is, a switching signal) from the control device is not input to the switching element 211, and becomes a state of hold. This allows automatically adjusting the resonance start timing in the resonant circuit 21.

Since other configurations and the advantage of the ninth embodiment are similar to the seventh embodiment, the further detailed description will be omitted.

### (Tenth Embodiment)

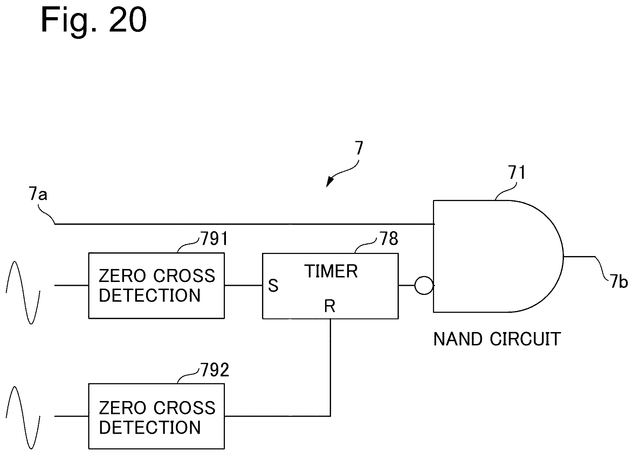

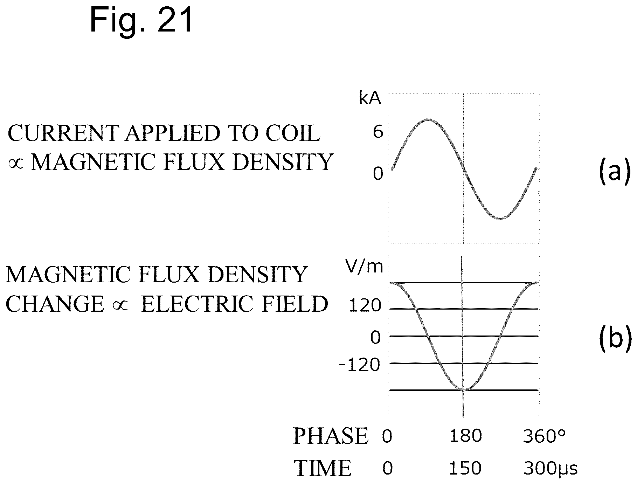

Next, a transcranial magnetic stimulator according to the tenth embodiment of the present invention will be described with reference to Fig. 20 and Fig. 21(a) and Fig. 21(b). In the description of the tenth embodiment, for the components basically in common with the device according to the seventh embodiment, the same reference numerals are used, thereby avoiding the overlapping description.

The tenth embodiment describes another example of the phase adjustment circuit 7 described in the seventh embodiment. The phase adjustment circuit 7 is configured to match the phases of the resonant currents by performing the adjustment such that maximum points of change rates (that is, dI/dt) of respective resonant currents generated in the resonant circuits 21, 22 are matched (that is, such that the difference is decreased).

The phase adjustment circuit 7 of the tenth embodiment includes a timer 78 connected to an inverting input of an AND gate 71, and zero cross detectors 791, 792 connected to the timer 78. The zero cross detector 791 is configured to detect a zero cross point in a sine waveform of the resonant current flowing in the resonant circuit 21. Similarly, the zero cross detector 792 is configured to detect a zero cross point in a sine waveform of the resonant current flowing in the resonant circuit 22. When the zero cross points are matched, an IGBT signal (that is, a switching signal) from the control device is directly input to the switching element 211. When the zero cross points are shifted, the shift is measured by the timer 78, and by the measured period, a time point of the next resonance start in one resonant circuit can be shifted (that is, delayed) by the time period. This allows matching the phases of the respective resonant currents generated in the resonant circuits 21, 22.

Fig. 21 illustrates a relation between the current applied to the coil and an electric field generated by the coil. The magnetic flux density of the coil is proportionate to the current value, and the electric field is proportionate to the change of the magnetic flux density. When the change rate (dI/dt) of the current applied to the coil is maximum, the electric field generated by the coil becomes maximum (see Fig. 21(b)). Accordingly, by matching the maximum points of the change rates of the currents, it can be attempted to make the electric field by a plurality of coils maximum. Therefore, a high treatment effect can be expected.

Since other configurations and the advantage of the tenth embodiment are similar to the seventh embodiment, the further detailed description will be omitted.

The contents of the present invention are not limited to the above-described embodiments. In the present invention, various kinds of changes can be made on the specific configurations within the scope of the claims.

For example, while the example of using the two resonant circuits is described in each of the above-described embodiments, it is possible to use three or more resonant circuits, thereby driving corresponding magnetic stimulation coils by the respective resonant circuits. In this case, the resonant circuits are each in parallel to the power source 3.

While the configuration in which the currents in the same phase are applied to the plurality of magnetic stimulation coils 11, 12 is described in each of the above-described embodiments, it is possible to apply a current in the opposite phase to invert the direction of the magnetic field, thereby canceling the magnetic field (ideally, making the magnetic field intensity zero). This allows the use as a sham stimulation coil for a clinical study.

### DESCRIPTION OF REFERENCE SIGNS

3...Power source
6... Synchronization adjustment circuit
7...Phase adjustment circuit
11...One magnetic stimulation coil (upper coil)
12... Other magnetic stimulation coil (lower coil)
21, 22...Resonant circuit
211, 221... Switching element
212, 222... Charging capacitor
213, 223...Resonant capacitor
214, 224...Diode
215, 225...Resistor
41, 42... Second switching element
51, 52...Resonant impedance circuit
100...Device main body
200... Adjustment mechanism
210... Coil holder

## Claims

1. A transcranial magnetic stimulator comprising:
a plurality of resonant circuits (21, 22) including a plurality of magnetic stimulation coils (11, 12) for stimulating a living body by applying variable magnetic fields to an inside of the living body, the plurality of resonant circuits (21, 22) applying respective pulse currents to the plurality of magnetic stimulation coils (11, 12) to generate the variable magnetic fields; and
a power source (3) that supplies an electric power to the plurality of resonant circuits (21, 22), wherein
the plurality of resonant circuits (21, 22) is connected in parallel to the power source (3), and therefore, the plurality of magnetic stimulation coils (11, 12) is also connected in parallel to the power source (3), and
the plurality of magnetic stimulation coils (11, 12) is formed in approximately a same shape, and adjacently disposed such that directions of magnetic fluxes generated by the pulse currents are matched;
wherein:
each of the plurality of resonant circuits (21, 22) includes a switching element (211, 221) that controls an application timing of the pulse current to the magnetic stimulation coil (11, 12).

2. The transcranial magnetic stimulator according to claim 1, wherein
the plurality of magnetic stimulation coils (11, 12) is disposed to be stacked such that axial centers of the plurality of magnetic stimulation coils are approximately matched.

3. The transcranial magnetic stimulator according to claim 2, wherein
one of the magnetic stimulation coils is an upper coil (11), and another is a lower coil (12), and
the upper coil (11) and the lower coil (12) are disposed to be stacked such that a bottom surface of the upper coil (11) is overlapped with an upper surface of the lower coil (12) in a cross-sectional view of at least a part of the upper coil (11) and the lower coil (12).

4. The transcranial magnetic stimulator according to claim 1, wherein
respective wound wires of the plurality of magnetic stimulation coils (11, 12) are mutually twisted, and form a multicore wire.

5. The transcranial magnetic stimulator according to any one of claims 1 to 4, wherein
each resonant circuit (21, 22) includes a resonant capacitor (213, 223) that accumulates an electric charge supplied from the power source (3),
a second switching element (41, 42) is disposed between the resonant capacitor (213, 223) and the power source (3), and the second switching element (41, 42) blocks a connection between the resonant capacitor (213, 223) and the power source (3) during discharge of the resonant capacitor (213, 223) to suppress a leakage current to the power source (3).

6. The transcranial magnetic stimulator according to any one of claims 1 to 4, wherein
the resonant circuit includes a resonant capacitor (213, 223) that accumulates an electric charge supplied from the power source, and
a resonant impedance circuit is interposed between the resonant capacitor (213, 223) and the power source, and the resonant impedance circuit acts as a resistance component higher than a resistance component in non-resonance by resonating at a resonant frequency of the resonant circuit to suppress a leakage current to the power source.

7. The transcranial magnetic stimulator according to any one of claims 1 to 6, wherein
any or all of the plurality of resonant circuits (21, 22) include a synchronization adjustment circuit for synchronizing the resonant frequency of each of the resonant circuits.

8. The transcranial magnetic stimulator according to any one of claims 1 to 7, comprising
a phase adjustment circuit (7) for matching phases of respective resonant currents generated in the plurality of resonant circuits (21, 22).

9. The transcranial magnetic stimulator according to claim 8, wherein
the phase adjustment circuit (7) is configured to match the phases of the resonant currents by performing an adjustment so as to match generation timings of the respective resonant currents generated in the plurality of resonant circuits (21, 22).

10. The transcranial magnetic stimulator according to claim 8, wherein
the phase adjustment circuit (7) is configured to match the phases of the resonant currents by performing an adjustment so as to match maximum points of change rates of the respective resonant currents generated in the plurality of resonant circuits (21, 22).

11. A transcranial magnetic stimulator comprising:
a plurality of resonant circuits (21, 22) including a plurality of magnetic stimulation coils (11, 12) for stimulating a living body by applying variable magnetic fields to an inside of the living body, the plurality of resonant circuits applying respective pulse currents to the plurality of magnetic stimulation coils (11, 12) to generate the variable magnetic fields; and
a power source (3) that supplies an electric power to the plurality of resonant circuits (21, 22), wherein
the plurality of resonant circuits is connected in parallel to the power source, and therefore, the plurality of magnetic stimulation coils (11, 12) is also connected in parallel to the power source, and
the transcranial magnetic stimulator further comprises a phase adjustment circuit (7) for matching phases of respective resonant currents generated in the plurality of resonant circuits (21, 22);
wherein:
each of the plurality of resonant circuits (21, 22) includes a switching element (211) that controls an application timing of the pulse current to the magnetic stimulation coil.

12. The transcranial magnetic stimulator according to claim 11, wherein
the phase adjustment circuit (7) is configured to match the phases of the resonant currents by performing an adjustment so as to match maximum points of change rates of the respective resonant currents generated in the plurality of resonant circuits (21, 22).

## Patentansprüche

1. Transkranieller Magnetstimulator, umfassend:
eine Vielzahl von Resonanzkreisen (21, 22), einschließlich einer Vielzahl von Magnetstimulationsspulen (11, 12) zum Stimulieren eines lebenden Körpers durch Anlegen veränderlicher Magnetfelder an das Innere des lebenden Körpers, wobei die Vielzahl von Resonanzkreisen (21, 22) jeweilige Impulsströme an die Vielzahl von Magnetstimulationsspulen (11, 12) anlegt, um die veränderlichen Magnetfelder zu erzeugen; und
eine Stromquelle (3), die der Vielzahl von Resonanzkreisen (21, 22) eine elektrische Leistung liefert, wobei
die Vielzahl von Resonanzkreisen (21, 22) parallel an die Stromquelle (3) geschaltet ist, und daher die Vielzahl von Magnetstimulationsspulen (11, 12) ebenfalls parallel an die Stromquelle (3) geschaltet ist, und
die Vielzahl von Magnetstimulationsspulen (11, 12) in annähernd derselben Form gebildet ist und benachbart zueinander so angeordnet ist, dass Richtungen von durch die Impulsströme erzeugten magnetischen Flüssen aufeinander abgestimmt sind;
wobei:
jeder der Vielzahl von Resonanzkreisen (21, 22) ein Schaltelement (211, 221) einschließt, das einen Anlegezeitpunkt des Impulsstroms an die Magnetstimulationsspule (11, 12) steuert.

2. Transkranieller Magnetstimulator nach Anspruch 1, wobei
die Vielzahl von Magnetstimulationsspulen (11, 12) zum Stapeln angeordnet ist, so dass die Achsmitten der Vielzahl von Magnetstimulationsspulen annähernd zusammenfallen.

3. Transkranieller Magnetstimulator nach Anspruch 2, wobei
eine der Magnetstimulationsspulen eine obere Spule (11) ist und eine andere eine untere Spule (12) ist, und
die obere Spule (11) und die untere Spule (12) so angeordnet sind, dass sie gestapelt sind, so dass eine Bodenfläche der oberen Spule (11) mit einer oberen Fläche der unteren Spule (12) in einer Querschnittsansicht von zumindest einem Teil der oberen Spule (11) und der unteren Spule (12) überlappt.

4. Transkranieller Magnetstimulator nach Anspruch 1, wobei
jeweilige Wicklungsdrähte der Vielzahl von Magnetstimulationsspulen (11, 12) miteinander verdrillt sind und einen mehradrigen Draht bilden.

5. Transkranieller Magnetstimulator nach einem der Ansprüche 1 bis 4, wobei
jeder Resonanzkreis (21, 22) einen Resonanzkondensator (213, 223) einschließt, der eine von der Stromquelle (3) gelieferte elektrische Ladung speichert,
ein zweites Schaltelement (41, 42) zwischen dem Resonanzkondensator (213, 223) und der Stromquelle (3) angeordnet ist, und das zweite Schaltelement (41, 42) während einer Entladung des Resonanzkondensators (213, 223) eine Verbindung zwischen dem Resonanzkondensator (213, 223) und der Stromquelle (3) blockiert, um einen Leckstrom zu der Stromquelle (3) zu unterdrücken.

6. Transkranieller Magnetstimulator nach einem der Ansprüche 1 bis 4, wobei
der Resonanzkreis einen Resonanzkondensator (213, 223) einschließt, der eine von der Stromquelle gelieferte elektrische Ladung speichert, und
eine Resonanzimpedanzschaltung zwischen dem Resonanzkondensator (213, 223) und der Stromquelle zwischengeschaltet ist, und die Resonanzimpedanzschaltung durch Resonieren bei einer Resonanzfrequenz des Resonanzkreises als eine Widerstandskomponente wirkt, die höher ist als eine Widerstandskomponente bei Nichtresonanz, um einen Leckstrom zu der Stromquelle zu unterdrücken.

7. Transkranieller Magnetstimulator nach einem der Ansprüche 1 bis 6, wobei
beliebige oder alle der Vielzahl von Resonanzkreisen (21, 22) eine Synchronisationsanpassungsschaltung zum Synchronisieren der Resonanzfrequenz jedes der Resonanzkreise einschließen.

8. Transkranieller Magnetstimulator nach einem der Ansprüche 1 bis 7, umfassend
eine Phasenanpassungsschaltung (7) zum Angleichen von Phasen jeweiliger Resonanzströme, die in der Vielzahl von Resonanzkreisen (21, 22) erzeugt werden.

9. Transkranieller Magnetstimulator nach Anspruch 8, wobei
die Phasenanpassungsschaltung (7) dazu ausgebildet ist, die Phasen der Resonanzströme durch Vornahme einer Anpassung anzugleichen, um Erzeugungszeitpunkte der jeweiligen Resonanzströme anzugleichen, die in der Vielzahl von Resonanzkreisen (21, 22) erzeugt werden.

10. Transkranieller Magnetstimulator nach Anspruch 8, wobei
die Phasenanpassungsschaltung (7) dazu ausgebildet ist, die Phasen der Resonanzströme durch Vornahme einer Anpassung anzugleichen, um Maximalpunkte der Änderungsraten der jeweiligen Resonanzströme anzugleichen, die in der Vielzahl von Resonanzkreisen (21, 22) erzeugt werden.

11. Transkranieller Magnetstimulator, umfassend:
eine Vielzahl von Resonanzkreisen (21, 22), einschließlich einer Vielzahl von Magnetstimulationsspulen (11, 12) zum Stimulieren eines lebenden Körpers durch Anlegen veränderlicher Magnetfelder an das Innere des lebenden Körpers, wobei die Vielzahl von Resonanzkreisen jeweilige Impulsströme an die Vielzahl von Magnetstimulationsspulen (11, 12) anlegt, um die veränderlichen Magnetfelder zu erzeugen; und
eine Stromquelle (3), die der Vielzahl von Resonanzkreisen (21, 22) eine elektrische Leistung liefert, wobei
die Vielzahl von Resonanzkreisen parallel an die Stromquelle geschaltet ist, und daher die Vielzahl von Magnetstimulationsspulen (11, 12) ebenfalls parallel an die Stromquelle geschaltet ist, und
der transkranielle Magnetstimulator ferner eine Phasenanpassungsschaltung (7) zum Angleichen von Phasen jeweiliger Resonanzströme umfasst, die in der Vielzahl von Resonanzkreisen (21, 22) erzeugt werden;
wobei:
jeder der Vielzahl von Resonanzkreisen (21, 22) ein Schaltelement (211) einschließt, das einen Anlegezeitpunkt des Impulsstroms an die Magnetstimulationsspule steuert.

12. Transkranieller Magnetstimulator nach Anspruch 11, wobei
die Phasenanpassungsschaltung (7) dazu ausgebildet ist, die Phasen der Resonanzströme durch Vornahme einer Anpassung anzugleichen, um Maximalpunkte der Änderungsraten der jeweiligen Resonanzströme anzugleichen, die in der Vielzahl von Resonanzkreisen (21, 22) erzeugt werden.

## Revendications

1. Stimulateur magnétique transcrânien comprenant :
une pluralité de circuits résonants (21, 22), incluant une pluralité de bobines de stimulation magnétique (11, 12) destinées à stimuler un corps vivant par application de champs magnétiques variables à l'intérieur du corps vivant, la pluralité de circuits résonants (21, 22) appliquant des courants pulsés respectifs à la pluralité de bobines de stimulation magnétique (11, 12) pour générer les champs magnétiques variables ; et
une source d'alimentation (3), qui fournit une puissance électrique à la pluralité de circuits résonants (21, 22), dans lequel
la pluralité de circuits résonants (21, 22) est connectée en parallèle à la source d'alimentation (3), et par conséquent, la pluralité de bobines de stimulation magnétique (11, 12) est aussi connectée en parallèle à la source d'alimentation (3), et
la pluralité de bobines de stimulation magnétique (11, 12) est formée dans approximativement une même forme, et disposée de manière adjacente de telle sorte que des directions de flux magnétiques générés par les courants pulsés soient en correspondance ;
dans lequel :
chaque circuit résonant de la pluralité de circuits résonants (21, 22) inclut un élément de commutation (211, 221) qui commande un instant d'application du courant pulsé à la bobine de stimulation magnétique (11, 12).

2. Stimulateur magnétique transcrânien selon la revendication 1, dans lequel
la pluralité de bobines de stimulation magnétique (11, 12) sont disposées de manière à être empilées de telle sorte que des centres axiaux des bobines de la pluralité de bobines de stimulation magnétique soient approximativement en correspondance.

3. Stimulateur magnétique transcrânien selon la revendication 2, dans lequel
l'une des bobines de stimulation magnétique est une bobine supérieure (11), et une autre est une bobine inférieure (12), et
la bobine supérieure (11) et la bobine inférieure (12) sont disposées de manière à être empilées de telle sorte qu'une surface inférieure de la bobine supérieure (11) soit superposée à une surface supérieure de la bobine inférieure (12) selon une vue en coupe d'au moins une partie de la bobine supérieure (11) et de la bobine inférieure (12).

4. Stimulateur magnétique transcrânien selon la revendication 1, dans lequel
des fils enroulés respectifs de la pluralité de bobines de stimulation magnétique (11, 12) sont mutuellement torsadés, et forment un fil multiconducteur.

5. Stimulateur magnétique transcrânien selon l'une quelconque des revendications 1 à 4, dans lequel
chaque circuit résonant (21, 22) inclut un condensateur de résonance (213, 223) qui accumule une charge électrique fournie à partir de la source d'alimentation (3),
un deuxième élément de commutation (41, 42) est disposé entre le condensateur de résonance (213, 223) et la source d'alimentation (3), et le deuxième élément de commutation (41, 42) bloque une connexion entre le condensateur de résonance (213, 223) et la source d'alimentation (3) pendant la décharge du condensateur de résonance (213, 223) pour supprimer un courant de fuite vers la source d'alimentation (3).

6. Stimulateur magnétique transcrânien selon l'une quelconque des revendications 1 à 4, dans lequel
le circuit résonant inclut un condensateur de résonance (213, 223) qui accumule une charge électrique fournie à partir de la source d'alimentation, et
un circuit d'impédance résonant est interposé entre le condensateur de résonance (213, 223) et la source d'alimentation, et le circuit d'impédance résonant agit comme une composante de résistance supérieure à une composante de résistance en non-résonance en entrant en résonance à une fréquence de résonance du circuit résonant pour supprimer un courant de fuite vers la source d'alimentation.

7. Stimulateur magnétique transcrânien selon l'une quelconque des revendications 1 à 6, dans lequel
un circuit résonant quelconque, ou la totalité des circuits résonants, de la pluralité de circuits résonants (21, 22) incluent un circuit de réglage de synchronisation pour synchroniser la fréquence de résonance de chacun des circuits résonants.

8. Stimulateur magnétique transcrânien selon l'une quelconque des revendications 1 à 7, comprenant
un circuit de réglage de phase (7) destiné à mettre en correspondance des phases de courants résonants respectifs générés dans la pluralité de circuits résonants (21, 22).

9. Stimulateur magnétique transcrânien selon la revendication 8, dans lequel
le circuit de réglage de phase (7) est configuré de manière à mettre en correspondance les phases des courants résonants en effectuant un réglage de sorte à mettre en correspondance des instants de génération des courants résonants respectifs générés dans la pluralité de circuits résonants (21, 22).

10. Stimulateur magnétique transcrânien selon la revendication 8, dans lequel
le circuit de réglage de phase (7) est configuré de manière à mettre en correspondance les phases des courants résonants en effectuant un réglage de sorte à mettre en correspondance des points maximaux de taux de variation des courants résonants respectifs générés dans la pluralité de circuits résonants (21, 22).

11. Stimulateur magnétique transcrânien comprenant :
une pluralité de circuits résonants (21, 22), incluant une pluralité de bobines de stimulation magnétique (11, 12) destinées à stimuler un corps vivant par application de champs magnétiques variables à l'intérieur du corps vivant, la pluralité de circuits résonants appliquant des courants pulsés respectifs à la pluralité de bobines de stimulation magnétique (11, 12) pour générer les champs magnétiques variables ; et
une source d'alimentation (3), qui fournit une puissance électrique à la pluralité de circuits résonants (21, 22), dans lequel
la pluralité de circuits résonants est connectée en parallèle à la source d'alimentation, et par conséquent, la pluralité de bobines de stimulation magnétique (11, 12) est aussi connectée en parallèle à la source d'alimentation, et
le stimulateur magnétique transcrânien comprend en outre un circuit de réglage de phase (7) destiné à mettre en correspondance des phases de courants résonants respectifs générés dans la pluralité de circuits résonants (21, 22) ;
dans lequel :
chaque circuit résonant de la pluralité de circuits résonants (21, 22) inclut un élément de commutation (211) qui commande un instant d'application du courant pulsé à la bobine de stimulation magnétique.

12. Stimulateur magnétique transcrânien selon la revendication 11, dans lequel
le circuit de réglage de phase (7) est configuré de manière à mettre en correspondance les phases des courants résonants en effectuant un réglage de sorte à mettre en correspondance des points maximaux de taux de variation des courants résonants respectifs générés dans la pluralité de circuits résonants (21, 22).
